(19) Europäisches Patentamt
European Patent Office
Office européen des brevets

(11) Veröffentlichungsnummer: **0 043 976**
**B1**

(12) # EUROPÄISCHE PATENTSCHRIFT

(45) Veröffentlichungstag der Patentschrift: **15.06.83**

(51) Int. Cl.³: **A 61 K 31/765**

(21) Anmeldenummer: **81105047.5**

(22) Anmeldetag: **30.06.81**

(54) **Wasserlösliche Polyester und Polycarbonate zur Verwendung als Antitumorwirkstoffe.**

(30) Priorität: **12.07.80 DE 3026447**

(43) Veröffentlichungstag der Anmeldung:
**20.01.82 Patentblatt 82/3**

(45) Bekanntmachung des Hinweises auf die Patenterteilung:
**15.06.83 Patentblatt 83/24**

(84) Benannte Vertragsstaaten:
**AT BE CH DE FR GB IT LI NL SE**

(56) Entgegenhaltungen:
**L. G. DONARUMA: »Polymeric drugs«, 1978, Seiten 240, 245 Academic Press New York, U.S.A.**

(73) Patentinhaber: **BAYER AG, Zentralbereich Patente, Marken und Lizenzen, D-5090 Leverkusen 1, Bayerwerk (DE)**

(72) Erfinder: **Wolf, Gerhard Dieter, Dr., Wilhelm-Busch-Strasse 29, D-4047 Dormagen 5 (DE)**
Erfinder: **Bömer, Bruno, Dr., Gustav-Freytag-Strasse 2, D-5090 Leverkusen 1 (DE)**
Erfinder: **Bierling, Robert, Dr., Merlinweg 18 b, D-5600 Wuppertal 1 (DE)**

**EP 0 043 976 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Wasserlösliche Polyester und Polycarbonate zur Verwendung als Antitumorwirkstoffe

Die vorliegende Erfindung betrifft die Verwendung von an sich bekannten wasserlöslichen Polyestern bzw. Polycarbonaten auf Basis von Polyetherdiolen als antitumoral wirkende Mittel.

Es ist schon bekanntgeworden, daß copolymere, komplexe $Cu^{II}$- und $Co^{II}$-Salze der Ethylenmaleinsäure gegen das Walker-Sarkom wirksam sind [J. Med. Chem. 12 (1969), 1180].

Weiterhin wurden Polykationen verschiedenen Typs, z. B. Polyamidamine, Poly-N-morpholinoethylacrylamid und N-Oxid-Polymere auf Hemmung der Metastasierung geprüft mit dem Ergebnis, daß nur die Tumorzellaussaat, nicht aber das Metastasenwachstum in situ bzw. Lymphknotenmetastasen beeinflußt werden konnten [J. Med. Chem. 16 (1973), 496].

Ferner wurde die Wirksamkeit von Polymeren mit Carboxylgruppen gegen das Sarkom 180 in Abhängigkeit vom Molekulargewicht, der Ladungsdichte, sowie der Metall-Bindungsfähigkeit der Carboxylgruppen beschrieben [Dissertation Abstr. Intern. B 33 (1973), 5745].

Polyanionen, z. B. Poly-ammoniumacrylat, Acrylsäureacrylamid-Copolymere sowie Copolymere aus Ethylen- u. Maleinsäureanhydrid sollen im Zusammenhang ihrer Antitumorwirkung einen Heparin-ähnlichen Effekt, daneben eine Virushemmung besitzen und außerdem die Immunreaktionen steigern [J. Med. Chem. 17 (1974), 1335].

Aus allen diesen Arbeiten geht hervor, daß die Antitumorwirkung der bisher untersuchten Polymeren an den verwendeten experimentellen Tumoren oft nur an der unteren Grenze der Signifikanz liegt und verschiedentlich nur auf prophylaktische oder adjuvantive Effekte beschränkt ist. Nachteilig erscheint außerdem, daß die zitierten Untersuchungen vielfach an allogenen, zur Spontanregression neigenden Mäusetumoren und nicht systematisch und unter kliniknahen Versuchsanordnungen durchgeführt wurden. Meist fehlen Angaben zur Toxizität der Präparate, obwohl die Applikation hoher Dosierungen von Substanzen mit einem Molekulargewicht von über 30 000 eine mangelhafte Ausscheidung bzw. Speicherung in den Geweben vermuten läßt.

W. Regelson et. al. [Nature 186 (1960) 778–880] untersuchten die Antitumorwirkung synthetischer Polyelektrolyte wie Polyacrylsäure, Polymethacrylsäure und hydrolysierten bzw. aminolysierten Ethylen-Maleinsäureanhydrid-Copolymerisaten. Sie fanden durch Wirkungsvergleich der Dicarbonsäure-, der Amid-Carbonsäure- und der Diamidform von Ethylen-Maleinsäureanhydrid-Copolymerisaten, daß für eine signifikante Tumorinhibierung mindestens eine ionisierbare Carboxylgruppe erforderlich ist. Versuche dieser Autoren mit Polyacrylamiden ergaben in hohen Dosen (800 mg/kg, MW 60–70 000 und 400 mg/kg, MW 120 000) eine negative, bzw. eine nicht signifikante positive Tumorhemmwirkung.

Ebenfalls wurden antitumoral wirkende Mittel beschrieben, die durch einen Gehalt an mindestens einem wasserlöslichen Homo- oder Copolymerisat, das das 1,3-Dihydroxy-2-methylenpropan und/oder dessen Derivate enthält, charakterisiert sind [DE-OS 2 705 189]. Ähnliche Präparate mit ähnlicher Wirkung sind wasserlösliche Homo- oder Copolymerisate, die 3,4-Dihydroxibuten-1 oder Hydroxi-alkyl-(meth)-acrylate sowie deren Derivate oder auch Derivate des Allylalkohols polymerisiert oder copolymerisiert enthalten [DE-OS 2 704 082].

Außerdem ist eine gewisse antitumorale Wirkung von Emulgatoren, die Polyethylenoxidketten eingebaut enthalten, bekanntgeworden. So wurde Polyoxiethylen-Sorbitan-monooleat (Tween 80) für die Immunisierung gegen den hyperdiploiden Ehrlich-Tumor verwendet [Experientia 29 (1973) 710].

Ein Blockcopolymerisat aus Poly-oxypropylen und Poly-oxyethylen (Pluronic F 68) erwies sich als wirksam gegen den Metastasenangang des Walker 256 Ascites Tumors, wahrscheinlich durch Beeinflussung der Blutgerinnungsfähigkeit [Cancer 29 (1972), 171]. Diese Präparate sind bekanntermaßen hoch wirksame Emulgatoren und sind aus diesem Grunde nicht sehr gut verträglich, insbesondere bei parenteraler Applikation.

Es wurde nun überraschenderweise gefunden, daß wasserlösliche Polyester und Polycarbonate, die aus Polyetherglycolen hergestellt worden sind, starke antitumorale Eigenschaften besitzen.

Die Substanzen zeigen im Molekulargewichtsbereich von etwa 1000 bis über 100 000 signifikante prophylaktische und kurative Wirkungen an soliden Tumoren syngener Systeme in einem breiten Dosisbereich von 0,5 bis 500 mg/kg, vorzugsweise 5 bis 250 mg/kg unter kliniknahen Versuchsanordnungen und Applikationsarten.

Die akute Toxizität gereinigter Substanzen ist gering; die LD 50 liegt bei i.v. Applikation meist oberhalb 2500 mg/kg, so daß die Substanzen eine ungewöhnlich große therapeutische Breite besitzen.

Die erfindungsgemäßen Wirkstoffe stellen somit eine wichtige Bereicherung der Therapie dar.

Die erfindungsgemäßen Wirkstoffe sind wasserlösliche Polyester bzw. Polycarbonate aus Polyetherglycolen, die der nachstehenden Formel entsprechen:

$$BO-\left[\left(\overset{\displaystyle A}{\overset{|}{C}}H-CH_2-O\right)_n-Q\right]_m-\left(\overset{\displaystyle A}{\overset{|}{C}}H-CH_2-O\right)_n-B$$

in welcher A statistisch verteilt H oder $CH_3$ ist, wobei maximal 50%, vorzugsweise weniger als 33% der A-Gruppen $CH_3$ sind;
die Gruppen B gleich oder verschieden sind und Wasserstoff, ein aliphatischer, alicyclischer oder aromatischer Rest mit 1 – 8 C-Atomen, vorzugsweise H oder $C_1-C_4$-Alkyl –,
eine Gruppe X – CO – mit X = Wasserstoff, aliphatischer, alicyclischer oder aromatischer Rest mit 1 – 7 C-Atomen, vorzugsweise H oder $C_1-C_3$-Alkyl –,
eine Gruppe Y – O – CO – mit Y = aliphatischer, cycloaliphatischer oder aromatischer Rest mit 1 – 7 C-Atomen, vorzugsweise $C_1-C_4$-Alkyl –
sowie eine Gruppe Z – NH – CO – mit Z = Wasserstoff, aliphatischer, cycloaliphatischer oder aromatischer Rest mit 1 – 7 C-Atomen, vorzugsweise H oder $C_1-C_4$-Alkyl – sind.
Besonders bevorzugt sind eine oder beide B-Gruppen Wasserstoff;

Q eine Gruppierung

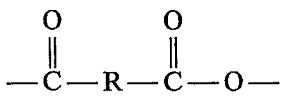 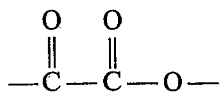

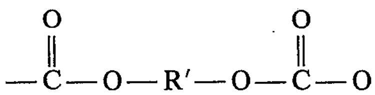 oder 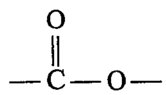

mit

R = Alkylen oder Arylen mit 1 – 20 C-Atomen, vorzugsweise 1 – 6 C-Atomen mit
R' = Alkylen mit 2 – 18 C-Atomen, vorzugsweise 2 – 6 C-Atomen

bedeutet;

n Zahlen von 5 – 500, vorzugsweise 8 – 300 und
m Zahlen von 1 – 30, vorzugsweise 1 – 15

bedeuten.

Beispiele für besonders bevorzugte Mittel gemäß der vorliegenden Erfindung sind folgende Verbindungen:

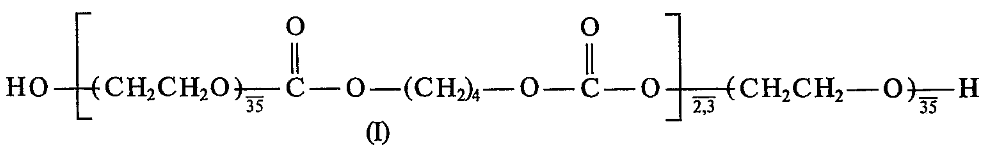

(I)

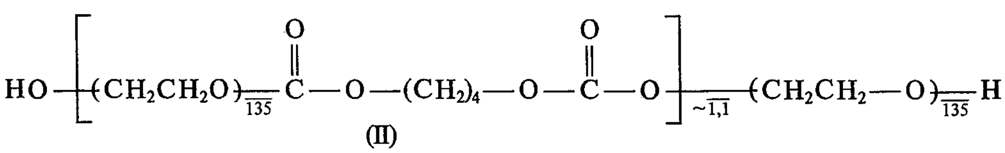

(II)

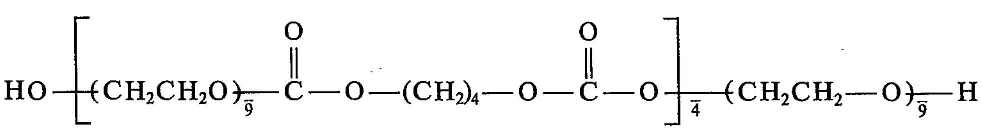

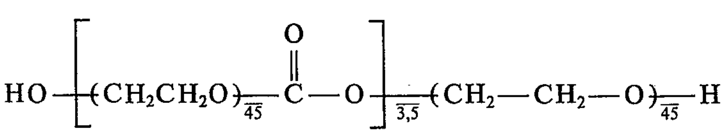

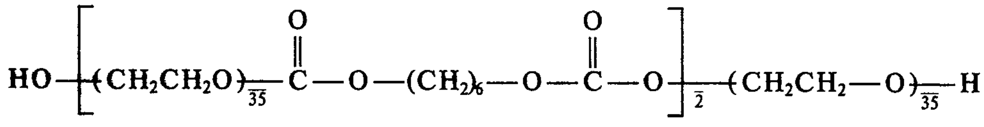

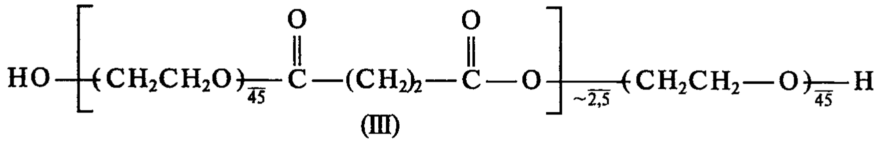

(III)

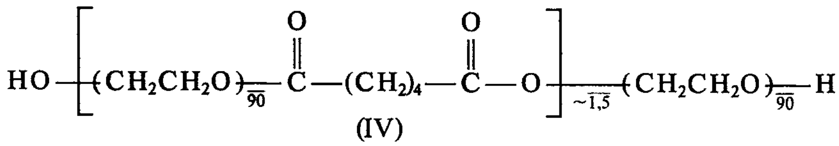

(IV)

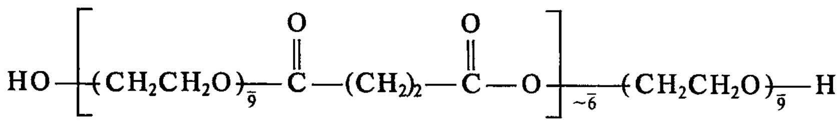

Die erfindungsgemäß zu verwendenden Polyester können nach bekannten Methoden hergestellt werden [Houben–Weyl, Methoden der organischen Chemie, 4. Auflage (1963), Bd. 14/II, S. 1 ff; Ullmanns Enzyklopädie der technischen Chemie, 3. Auflage, Bd. 14 (1963), S. 80–85].

Besonders bevorzugt ist die Herstellung durch azeotrope Kondensation oder durch Lösungskondensation, wobei man von einem Polyetherglykol und einer Dicarbonsäure ausgeht, die Veresterung in Gegenwart eines sauren Katalysators durchführt und das gebildete Reaktionswasser azeotrop, z. B. mit Toluol abdestilliert. Der Polymerisationsgrad m richtet sich nach dem Molverhältnis Diol zu Dicarbonsäure und dem Grad der Umsetzung (Vollständigkeit der Umsetzung). Arbeitet man mit einem molaren Überschuß an Polyetherglykol, so erhält man Polyester mit überwiegend OH-Endgruppen. Selbstverständlich kann man außer Dicarbonsäuren auch Dicarbonsäuredichloride verwenden und diese in Gegenwart tert. Amine mit den Polyetherglykolen umsetzen.

Die erfindungsgemäß zu verwendenden Polycarbonate können entweder durch Phosgenierung in wasserfreiem Medium in Gegenwart einer indifferenten Base, oder durch Umsetzung eines Bis-chlorameisensäureesters einer niedermolekularen Dihydroxyverbindung oder eines Polyetherglykols mit einem Polyetherglykol in Gegenwart einer indifferenten Base hergestellt werden [Houben–Weyl, Methoden der organischen Chemie, 4. Auflage, Bd. 14/II (1963), S. 53 und 54]. Auch in diesem Falle hängt der Polykondensationsgrad m vom Molverhältnis Diol zu Phosgen, bzw. Bis-Chlorameisensäureester ab.

Es können selbstverständlich auch Gemische von Polyetherglykolen verschiedener mittlerer Molekulargewichte sowie Gemische von 2 oder mehr Dicarbonsäuren sowie von 2 oder mehr Bis-chlorameisensäureestern zur Herstellung erfindungsgemäß zu verwendender Polyester bzw. Polycarbonate verwendet werden.

Die Hydroxylendgruppen der Polyester oder Polycarbonate können nach bekannten Methoden in Ethergruppen, Carbonsäureestergruppen, Kohlensäureestergruppen oder Urethangruppen überführt werden. Bei der Polyesterherstellung können Esterendgruppen durch Mitverwendung entsprechender Mengen einer Monocarbonsäure und bei der Polycarbonatherstellung durch Zusatz eines Chlorameisensäureesters eines Monoalkohols eingeführt werden.

Die zur Herstellung der erfindungsgemäß zu verwendenden Polyester oder Polycarbonate benötigten Polyetherdiole sind dem Fachmann bekannt. Sie werden in großen Mengen zur Herstellung von Polyurethankunststoffen verwendet. Ihre Herstellung erfolgt durch ringöffnende Polymerisation oder Copolymerisation von Ethylenoxid und Propylenoxid (Houben–Weyl, Methoden der org. Chemie, 4. Auflage, Bd. XIV/2, 1963, S. 427 ff., Ullmanns Encyklopädie der technischen Chemie, 3. Auflage, Bd. 14 (1963), S. 49–52). Ausgehend von Ethylenoxid erhält man die Polyethylenglykole, die vom Diethylenglykol bis zu Produkten mit Molekulargewichten über $10^6$ sehr gut in Wasser löslich sind. Von den aus Propylenoxid entstehenden Polypropylenglykolen sind nur die relativ niedermolekularen Vertreter in Wasser löslich. Die Wasserlöslichkeit der Polypropylenglykole sowie der Copolymerisate von Ethylenoxid und Propylenoxid kann durch den Einbau ionischer Gruppen verbessert werden. Statistische Copolymere von Ethylenoxid und Propylenoxid sind je nach ihrem Molekulargewicht und Gehalt an Propylenoxidbausteinen mehr oder weniger gut in Wasser löslich.

Die erfindungsgemäßen Wirkstoffe können in physiologischer Kochsalzlösung bei Temperaturen von 20–40°C zu mindestens 0,5gewichtsprozentigen Lösungen aufgelöst werden. Sie besitzen bei äußerst niedriger Toxizität eine starke antitumorale Wirkung gegen tierische und menschliche

Tumoren und sollen daher zur Bekämpfung von durch Tumoren verursachten Erkrankungen verwendet werden.

Die erfindungsgemäßen Wirkstoffe werden zur Anwendung in physiologischer Kochsalzlösung gelöst. Sie können aber auch in Tabletten, Kapseln, Säfte und andere übliche Zubereitungsformen für die orale Applikation eingearbeitet werden. Die dabei erhaltenen Zubereitungen werden intraperitoneal, intravenös, intramuskulär oder oral appliziert.

Der therapeutische Dosisbereich reicht von 0,5 bis 500 mg/kg, vorzugsweise 5 bis 250 mg/kg. Die ungewöhnliche Breite dieses Bereiches beruht auf der ungewöhnlichen Ungiftigkeit der Wirkstoffe.

Die Substanzen wurden in zahlreichen Versuchen unter verschiedenen Testbedingungen am Carcinom EO 771 bzw. am Mäusesarkom MCS 4 auf die Induktion von Tumorwirkungen geprüft.

Die Methodik der Untersuchungen an diesen beiden experimentellen Tumoren ist aus den Versuchsbeschreibungen (a) und (b) ersichtlich.

Versuchsbeschreibung a)

Tumor-Teste am Carcinom EO 771 auf C 57 BL/6-Mäusen

Tierart:
C 57 BL/6-Mäuse, Inzucht (SPF)

Verfahren

Stammhaltung:
Am 14. bis 20. Tag nach letzter Transplantation subcutane Verimpfung einer Zellsuspension des Carcinoms EO 771 in 0,5 ml Phosphat-gepufferter 0,9%iger NaCl-Lösung auf C 57 BL/6-Mäuse.

Vorbereitung von Screening-Testen:
Gleiches Verfahren wie bei Stammhaltung des Tumors, jedoch subcutane Verimpfung einer Suspension von $5 \times 10^4$ Tumorzellen in 0,5 ml Phosphat-gepufferter 0,9%iger NaCl-Lösung.

Behandlung:
Einmalige, intramuskuläre Injektion der geforderten Präparatlösungen am 6. Tag vor bzw. am 2. Tag nach der Tumortransplantation.

Versuchsdauer:
18–22 Tage nach der Tumortransplantation. Dann Abtötung der Tiere, Präparation und Wägung der subcutanen Tumoren.

Auswertungsparameter:
Hemmung des Tumorwachstums durch Bestimmung des durchschnittlichen Tumorgewichts von Kontrollen und behandelten Tiergruppen sowie Errechnung des Tumorgewichts-(TG-)-Index nach der Formel:

$$\text{Tg-Index} = \frac{\Phi \text{ Tumorgewicht der behandelten Tiergruppen}}{\Phi \text{ Tumorgewicht der Kontrollgruppe}}.$$

Beurteilung der Testergebnisse

TG-Index
0,8–0,6 = Wirkungsandeutung;
0,6–0,4 = deutliche Wirkung;
<0,4 = gute Wirkung.

Versuchsbeschreibung b)

Tumor-Teste am Sarkom MCS 4 auf C 57 BL/6-Mäusen

Stammhaltung:
10–14 Tage nach letzter Transplantation subcutane Verimpfung einer Tumorsuspension des Sarkoms MCS 4 in 0,5 ml einer Phosphat-gepufferten 0,9%igen NaCl-Lösung auf C 57 BL/6-Mäuse.

Vorbereitung von Screening-Testen:

Gleiches Verfahren wie bei Stammhaltung des Tumors, jedoch subcutane Verimpfung einer Suspension von $2 \times 10^5$ Tumorzellen in 0,5 ml einer Phosphat-gepufferten 0,9%igen NaCl-Lösung.

Behandlung:

Einmalige, intravenöse Injektion der geforderten Präparatlösung am 2. Tag vor bzw. am 2. Tag nach der Tumortransplantation.

Versuchsdauer:

18–22 Tage nach der Tumortransplantation. Dann Abtötung der Tiere, Präparation und Wägung der Tumoren.

Auswertung und Beurteilung der Ergebnisse erfolgen analog wie in der Versuchsbeschreibung a).

Tabelle 1

Prüfergebnisse am Carcinom EO 771

| Verbindung Nr. | | Dosis mg/kg | Applikation 1 × | Behandlungstag*) | Tumorgewichtsindex |
|---|---|---|---|---|---|
| I | (Beispiel 1) | 250 | i. m. | –6 | 0,36 |
| | | 250 | i. m. | +2 | 0,38 |
| II | (Beispiel 2) | 50 | i. m. | –6 | 0,39 |
| | | 50 | i. m. | +2 | 0,43 |
| III | (Beispiel 3) | 250 | i. m. | –6 | 0,23 |
| | | 250 | i. m. | +2 | 0,42 |

*) Behandlungstag –6 = 6 Tage vor Tumortransplantation

+2 = 2 Tage nach Tumortransplantation

Tabelle 2

Prüfergebnisse am Sarkom MCS 4

| Verbindung Nr. | | Dosis mg/kg | Applikation 1 × | Behandlungstag*) | Tumorgewichtsindex |
|---|---|---|---|---|---|
| I | (Beispiel 1) | 0,5 | i. v. | −2 | 0,45 |
| | | 5 | i. v. | +2 | 0,39 |
| III | (Beispiel 3) | 50 | i. v. | −2 | 0,24 |
| | | 50 | i. v. | +2 | 0,32 |
| IV | (Beispiel 4) | 2,5 | i. v. | −2 | 0,37 |
| | | 10 | i. v. | +2 | 0,32 |

*) Behandlungstag −2 = 2 Tage vor Tumortransplantation

+2 = 2 Tage nach Tumortransplantation

Die Tumorgewichts-Indices der aufgeführten Präparate in den Tabellen 1 und 2 lassen erkennen, daß die Substanzen sowohl am Carcinom EO 771, als auch am Sarkom MCS 4 bei verschiedenen Dosierungen und Applikationswegen, sowie an verschiedenen Behandlungstagen deutliche Tumorhemmwirkungen zu induzieren vermögen.

Beispiel 1

200 g Polyethylenglykol mit einem mittleren Molekulargewicht $\overline{M}_n$ von 1550 werden in 1 l Toluol gelöst. Zum Trocknen werden 200 ml Toluol unter Normaldruck abdestilliert. Nach dem Abkühlen auf Raumtemperatur werden 20 g trockenes Pyridin zugegeben und anschließend werden unter Rühren 20 g Butandiol-bis-chlorameisensäureester zugetropft und die Reaktionsmischung 3 Stunden bei Raumtemperatur nachgerührt. Das Pyridinhydrochlorid wird abgesaugt, gründlich mit Toluol gewaschen und die Toluollösung am Rotationsverdampfer im Vakuum eingedampft. Der Rückstand wird zweimal aus Ethylacetat/Diethylether 2 : 1 umkristallisiert, mit Diethylether nachgewaschen und im Vakuum getrocknet.

Ausbeute: 180 g, $\overline{M}_n = 5200$ (bestimmt durch Membranosmometrie in DMF).

Beispiel 2

200 g Polyethylenglykol mit einem Molekulargewicht $\overline{M}_n = 6000$ werden in 1 l Toluol gelöst und durch Abdestillieren von 200 ml Toluol getrocknet. Nach dem Abkühlen auf Raumtemperatur werden 4 g trockenes Pyridin zugegeben und eine Lösung von 3,6 g Butandiol-bis-chlorameisensäureester in 50 ml Toluol zugetropft. Die Reaktionsmischung wird über Nacht bei Raumtemperatur gerührt. Der Ansatz wird dann mit 1 l Toluol verdünnt und 1 Stunde weitergerührt. Anschließend wird das Polycarbonat nach dem Abtrennen des Pyridinhydrochlorids wie in Beispiel 1 beschrieben isoliert.

Ausbeute: 185 g, $\overline{M}_n = 1{,}35 \times 10^4$ (bestimmt durch Membranosmometrie in DMF).

Beispiel 3

200 g Polyethylenglykol mit einem Molekulargewicht $\overline{M}_n = 2000$, 8,85 g Bernsteinsäure und 1 g p-Toluolsulfonsäure werden in 500 ml Toluol gelöst. Unter Feuchtigkeitsausschluß wird Toluol mit ca. 50 ml/h abdestilliert. Sobald 150 ml destilliert sind, werden 150 ml absolutes Toluol zugegeben. Sobald kein Wasser mehr übergeht (nach ca. 12 Stunden), wird das Toluol am Rotationsverdampfer im

Vakuum entfernt und der Rückstand zweimal aus Ethylacetat/Diethylether 2 : 1 umkristallisiert.
Ausbeute: 177 g, $\overline{M}_n$ = 7300 (bestimmt durch Membranosmometrie in DMF).

Beispiel 4

250 g Polyethylenglykol mit einem Molekulargewicht $\overline{M}_n$ = 4000, 6,1 g Adipinsäure und 1 g p-Toluolsulfonsäure werden in 500 ml Toluol gelöst. Analog zu Beispiel 3 wird 24 h lang Toluol abdestilliert und der gebildete Polyester durch zweifaches Umkristallisieren gereinigt.
Ausbeute: 227 g, $\overline{M}_n$ = 10 500 (bestimmt durch Membranosmometrie in DMF).

**Patentansprüche**

1. Wasserlösliche Polyester und Polycarbonate auf Basis von Polyetherdiolen entsprechend der allgemeinen Formel

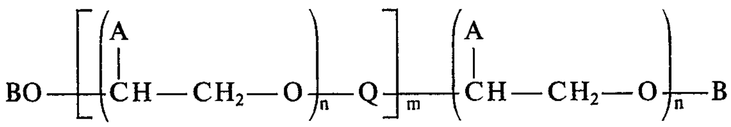

in welcher A statistisch verteilt H oder $CH_3$ ist, wobei maximal 50% der A-Gruppen $CH_3$ sind;
die Gruppen B gleich oder verschieden sind und Wasserstoff, ein aliphatischer, alicyclischer oder aromatischer Rest mit 1 – 8 C-Atomen,
eine Gruppe X – CO – mit X = Wasserstoff, aliphatischer, alicyclischer oder aromatischer Rest mit 1 – 7 C-Atomen,
eine Gruppe Y – O – CO – mit Y = aliphatischer, cycloaliphatischer oder aromatischer Rest mit 1 – 7 C-Atomen
sowie eine Gruppe Z – NH – CO – mit Z = Wasserstoff, aliphatischer, cycloaliphatischer oder aromatischer Rest mit 1 – 7 C-Atomen sind,

Q eine Gruppierung

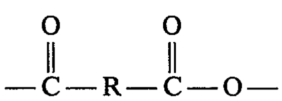 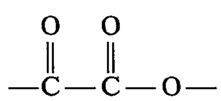

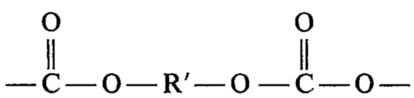 oder 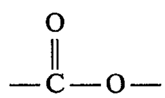

mit

R = Alkylen oder Arylen mit 1 – 20 C-Atomen und
R′ = Alkylen mit 2 – 18 C-Atomen

bedeutet;

n Zahlen von 5 – 500 und
m Zahlen von 1 – 30

bedeuten zur Verwendung als Antitumorwirkstoffe.

2. Wasserlösliche Polyester auf der Basis von Polyetherglykolen entsprechend der allgemeinen Formel

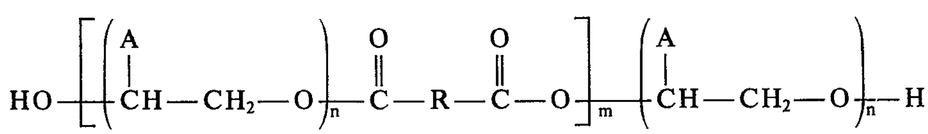

in welcher A statistisch verteilt H oder $CH_3$, mit 67 – 100 Mol% H und 0 – 33 Mol% $CH_3$,

R = Alkylen oder Arylen mit 1 – 6 C-Atomen,
n = eine Zahl von 8 – 300 und
m = eine Zahl von 1 – 15

bedeuten, zur Verwendung als Antitumorwirkstoffe.

3. Wasserlösliche Polycarbonate auf der Basis von Polyetherglykolen der allgemeinen Formel

$$HO-\left[\left(\overset{\displaystyle A}{\overset{|}{C}}H-CH_2-O\right)_n-\overset{\displaystyle O}{\overset{\|}{C}}-O\right]_m-\left(\overset{\displaystyle A}{\overset{|}{C}}H-CH_2-O\right)_n-H$$

in welcher A, n und m die gleiche Bedeutung wie in Anspruch 2 besitzen, zur Verwendung als Antitumorwirkstoffe.

4. Wasserlösliche Polycarbonate auf der Basis von Polyetherglykolen der allgemeinen Formel

$$HO-\left[\left(\overset{\displaystyle A}{\overset{|}{C}}H-CH_2-O\right)_n-\overset{\displaystyle O}{\overset{\|}{C}}-O-R'-O-\overset{\displaystyle O}{\overset{\|}{C}}-O\right]_m-\left(\overset{\displaystyle A}{\overset{|}{C}}H-CH_2-O\right)_n-H$$

in welcher

A statistisch verteilt H oder $CH_3$ bedeutet mit maximal 33 Mol% $CH_3$;
R' eine Alkylengruppe mit 2 – 6 C-Atomen,
n eine Zahl von 8 – 300 und
m eine Zahl von 1 – 15

bedeutet, zur Verwendung als Antitumorwirkstoffe.

5. Wasserlösliche Polyester auf Basis von Polyethylenglykolen entsprechend der allgemeinen Formel

$$HO-\left[(CH_2-CH_2-O)_n-\overset{\displaystyle O}{\overset{\|}{C}}-R-\overset{\displaystyle O}{\overset{\|}{C}}-O\right]_m-(CH_2-CH_2-O)_n-H$$

in welcher

n Zahlen von 8 – 300,
m Zahlen von 1 – 15 und
R Alkylen oder Arylen mit 1 – 20 C-Atomen

bedeutet, zur Verwendung als Antitumorwirkstoffe.

6. Wasserlösliche Polycarbonate auf Basis von Polyethylenglykolen entsprechend der allgemeinen Formel

$$HO-\left[(CH_2-CH_2-O)_n-\overset{\displaystyle O}{\overset{\|}{C}}-O-R'-O-\overset{\displaystyle O}{\overset{\|}{C}}-O\right]_m-(CH_2-CH_2-O)_n-H$$

in welcher

n Zahlen von 8 – 300,
m Zahlen von 1 – 15 und
R' Alkylen mit 1 – 6 C-Atomen

bedeutet, zur Verwendung als Antitumorwirkstoffe.

7. Wasserlösliche Polycarbonate auf Basis von Polyethylenglykolen entsprechend der allgemeinen Formel

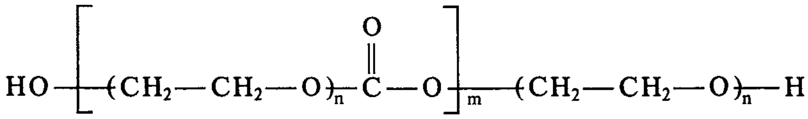

in welcher

n Zahlen von 8 – 300 und
m Zahlen von 1 – 15

bedeuten, zur Verwendung als Antitumorwirkstoffe.

8. Verfahren zur Herstellung eines antitumoral wirkenden Mittels, dadurch gekennzeichnet, daß man mindestens einen wasserlöslichen Polyester bzw. ein wasserlösliches Polycarbonat aus Polyetherglykolen gemäß den Ansprüchen 1 – 7 in einer physiologischen Kochsalzlösung löst oder in Zubereitungen für die orale Applikation einarbeitet.

## Claims

1. Water-soluble polyesters and polycarbonates based on polyether diols, corresponding to the general formula

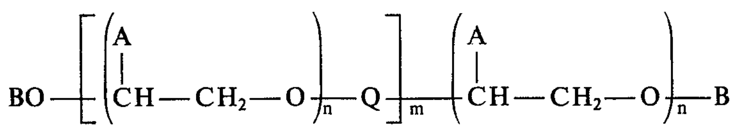

in which

A is statistically distributed H or $CH_3$, a maximum of 50% of the A groups being $CH_3$, the groups B are identical or different and are hydrogen, an aliphatic, alicyclic or aromatic radical having 1 – 8 C atoms, a group X – CO –, X being hydrogen or an aliphatic, alicyclic or aromatic radical having 1 – 7 C atoms, a group Y – O – CO –, Y being an aliphatic, cycloaliphatic or aromatic radical having 1 – 7 C atoms, or also a group Z – NH – CO –, Z being hydrogen or an aliphatic, cycloaliphatic or aromatic radical having 1 – 7 C atoms,

Q denotes a group

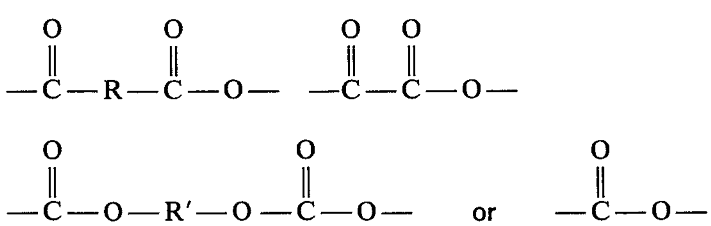

R being alkylene or arylene having 1 – 20 C atoms and
R' being alkylene having 2 – 18 C atoms;
n denotes numbers from 5 – 500 and
m denotes numbers from 1 – 30,

for use as tumour-inhibiting active compounds.

2. Water-soluble polyesters based on polyether-glycols, corresponding to the general formula

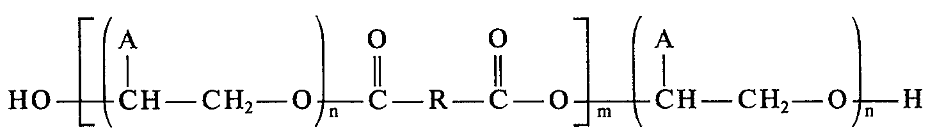

in which

A denotes statistically distributed H or $CH_3$, 67 – 100 mol % being H and 0 – 33 mol % being $CH_3$,
R denotes alkylene or arylene having 1 – 6 C atoms,
n denotes a number from 8 – 300 and
m denotes a number from 1 – 15,

for use as tumour-inhibiting active compounds.

3. Water-soluble polycarbonates based on polyether-glycols, of the general formula

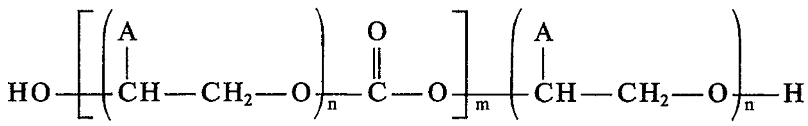

in which

A, n and m have the same meanings as in Claim 2,

for use as tumour-inhibiting active compounds.

4. Water-soluble polycarbonates based on polyether-glycols, of the general formula

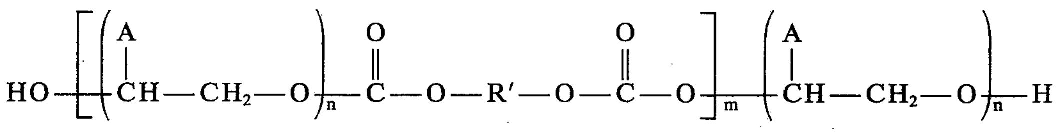

in which

A denotes statistically distributed H or $CH_3$, a maximum of 33 mol % being $CH_3$,

R′ denotes an alkylene group having 2 – 6 C atoms,
n denotes a number from 8 – 300 and
m denotes a number from 1 – 15,

for use as tumour-inhibiting active compounds.

5. Water-soluble polyesters based on polyethylene glycols, corresponding to the general formula

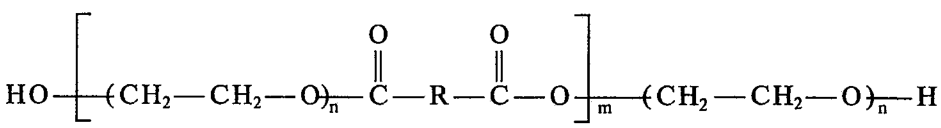

in which

n denotes numbers from 8 – 300,
m denotes numbers from 1 – 15 and
R denotes alkylene or arylene having 1 – 20 C atoms,

for use as tumour-inhibiting active compounds.

6. Water-soluble polycarbonates based on polyethylene glycols, corresponding to the general formula

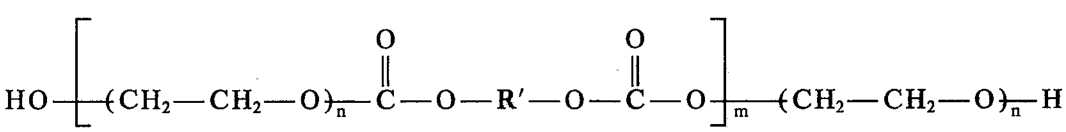

in which

n denotes numbers from 8 – 300,
m denotes numbers from 1 – 15 and
R′ denotes alkylene having 1 – 6 C atoms,

for use as tumour-inhibiting active compounds.

7. Water-soluble polycarbonates based on polyethylene glycols, corresponding to the general formula

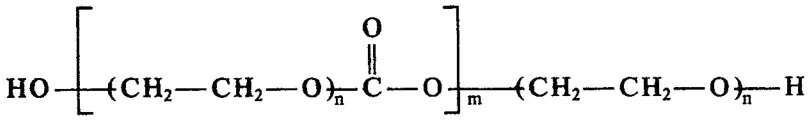

in which

n denotes numbers from 8–300 and
m denotes numbers from 1–15,

for use as tumour-inhibiting active compounds.

8. Process for the preparation of an agent having a tumour-inhibiting action, characterised in that at least one water-soluble polyester or one water-soluble polycarbonate from polyether-glycols, according to Claims 1–7, is dissolved in a physiological sodium chloride solution or incorporated in preparations for oral administration.

**Revendications**

1. Polyesters et polycarbonates hydrosolubles à base de polyéther-diols répondant à la formule générale

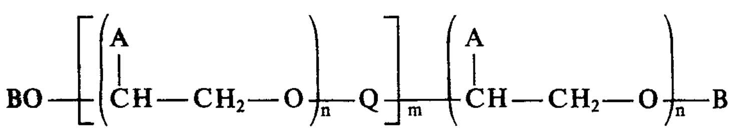

dans laquelle A représente l'hydrogène ou le groupe méthyle réparti statistiquement, 50% au maximum des groupes A étant des groupes $CH_3$;
les groupes B sont égaux ou différents et représentent l'hydrogène, un reste aliphatique, alicyclique ou aromatique ayant 1 à 8 atomes de carbone,
un groupe X–CO– dans lequel X = hydrogène, reste aliphatique, alicyclique ou aromatique ayant 1 à 7 atomes de carbone,
un groupe Y–O–CO– dans lequel Y = reste aliphatique, cycloaliphatique ou aromatique ayant 1 à 7 atomes de carbone
ainsi qu'un groupe Z–NH–CO– dans lequel Z = hydrogène, reste aliphatique, cycloaliphatique ou aromatique ayant 1 à 7 atomes de carbone,

Q représente un groupement

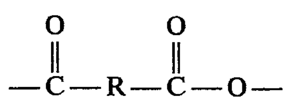
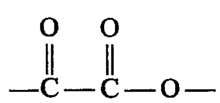

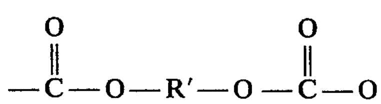 ou 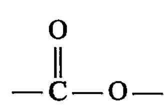

avec

R = alkylène ou arylène ayant 1–20 atomes de carbone et
R' = alkylène ayant 2–18 atomes de carbone;
n représente des nombres de 5–500 et
m représente des nombres de 1–30

destinés à être utilisés comme substances actives anti-tumorales.

2. Polyesters hydrosolubles à base de polyéther-glycols répondant à la formule générale

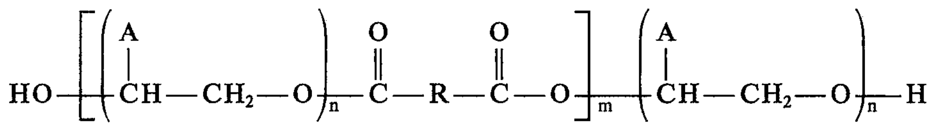

dans laquelle A représente H ou le groupe méthyle à répartition statistique, avec 67 – 100 moles % de H et 0 – 33 moles % de $CH_3$,

R = alkylène ou arylène ayant 1 – 6 atomes de carbone,
n = un nombre de 8 – 300 et
m = un nombre de 1 – 15,

destinés à être utilisés comme substances actives anti-tumorales.

3. Polycarbonates hydrosolubles à base de polyéther-glycols de formule générale

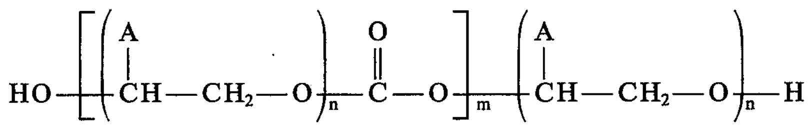

dans laquelle A, n et m ont la même définition que dans la revendication 2, destinés à être utilisés comme substances actives anti-tumorales.

4. Polycarbonates hydrosolubles à base de polyéther-glycols de formule générale

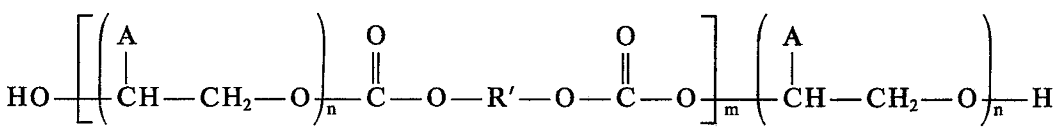

dans laquelle

A désigne H ou le groupe méthyle à répartition statistique avec un maximum de 33 moles % de $CH_3$;
R′ est un groupe alkylène ayant 2 – 6 atomes de carbone,
n est un nombre de 8 – 300 et
m est un nombre de 1 – 15,

destinés à être utilisés comme substances actives anti-tumorales.

5. Polyesters hydrosolubles à base de polyéthylèneglycols répondant à la formule

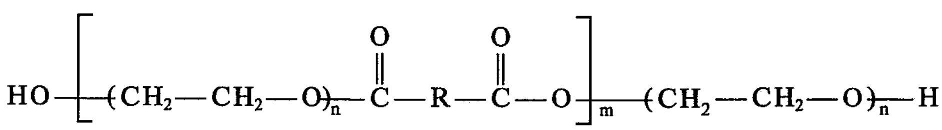

dans laquelle

n désigne des nombres de 8 à 300,
m désigne des nombres de 1 à 15 et
R est un reste alkylène ou arylène ayant 1 – 20 atomes de carbones, destinés à être utilisés comme substances actives anti-tumorales.

6. Polycarbonates hydrosolubles à base de polyéthylèneglycols répondant à la formule générale

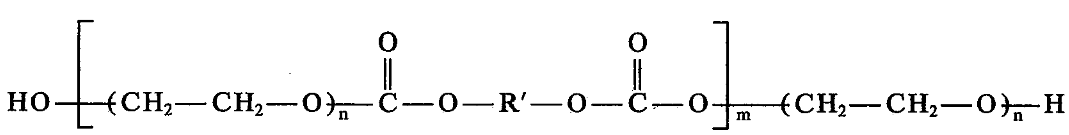

dans laquelle

n désigne des nombres de 8 à 300,
m désigne des nombres de 1 à 15 et

R' est un groupe alkylène ayant 1—6 atomes de carbone, destinés à être utilisés comme substances actives anti-tumorales.

7. Polycarbonates hydrosolubles à base de polyéthylèneglycols répondant à la formule générale

$$HO\!\left[\!-\!(CH_2-CH_2-O)_n\!-\!\overset{\overset{\displaystyle O}{\|}}{C}\!-\!O\!-\!\right]_m\!-\!(CH_2-CH_2-O)_n\!-\!H$$

dans laquelle

n représente des nombres de 8 à 300 et
m représente des nombres de 1 à 15

destinés à être utilisés comme substances actives anti-tumorales.

8. Procédé de production d'une composition à action anti-tumorale, caractérisé en ce qu'on dissout au moins un polyester hydrosoluble ou un polycarbonate hydrosoluble à base de polyéther-glycols suivant les revendications 1—7 dans une solution physiologique de chlorure de sodium, ou on l'incorpore dans des préparations destinées à l'administration orale.